(19) Europäisches Patentamt
European Patent Office
Office européen des brevets

(11) Veröffentlichungsnummer: **0 173 948**
**A2**

(12) # EUROPÄISCHE PATENTANMELDUNG

(21) Anmeldenummer: **85110753.2**

(22) Anmeldetag: **27.08.85**

(51) Int. Cl.[4]: **C 07 H 15/22**
C 07 H 3/06, C 12 P 19/12
C 12 P 19/28, A 61 K 31/70
//(C12P19/28, C12R1:465)

(30) Priorität: **04.09.84 DE 3432431**

(43) Veröffentlichungstag der Anmeldung:
**12.03.86 Patentblatt 86/11**

(84) Benannte Vertragsstaaten:
**AT BE CH DE FR GB IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Vértesy, László, Dr.**
**Eppenhainer Weg 6**
**D-6239 Eppstein/Taunus(DE)**

(72) Erfinder: **Bender, Rudolf, Dr.**
**Viktoriastrasse 6**
**D-6242 Kronberg/Taunus(DE)**

(72) Erfinder: **Fehlhaber, Hans-Wolfram, Dr.**
**Thomas-Mann-Strasse 5a**
**D-6270 Idstein/Taunus(DE)**

(72) Erfinder: **Geisen, Karl, Dr.**
**Jahnstrasse 43**
**D-6000 Frankfurt am Main 1(DE)**

(54) **Neue Pseudooligosaccharide mit alpha-Glukosidase-hemmender Wirkung, Verfahren zur deren Herstellung, deren Verwendung und pharmazeutische Präparate.**

(57) Pseudooligosaccharide der Formel I

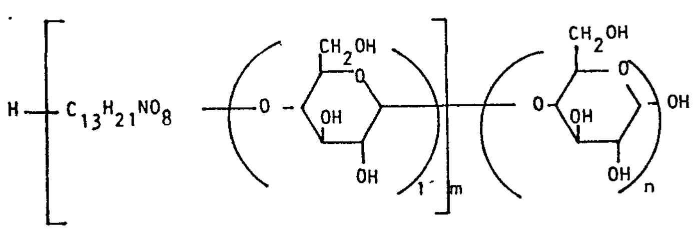

I

worin 1, m und n die angegebenen Bedeutungen haben, deren physiologisch verträgliche Slaze mit Säuren, Verfahren zu ihrer Herstellung, pharmazeutische Präparate und ihre Verwendung werden beschrieben. Die Verbindungen besitzen α-Glukosidase-hemmende Wirkung.

EP 0 173 948 A2

Croydon Printing Company Ltd.

HOECHST AKTIENGESELLSCHAFT HOE 84/F 207 Dr.D/Sr 0173948

# Neue Pseudooligosaccharide mit α-Glukosidase-hemmender Wirkung, Verfahren zu deren Herstellung, deren Verwendung und pharmazeutische Präparate

Die Erfindung betrifft neue, biologisch aktive Pseudooligosaccharide und ihre physiologisch verträglichen Salze. Sie besitzen α-Glukosidase-, d.h. z.B. α-Amylase- und Disaccharidase-hemmende Eigenschaften und können daher in der Human- und Tiermedizin, in der Tierernährung sowie in der Biotechnologie der Stärke verwendet werden.

Die erfindungsgemäßen Pseudooligosaccharide weisen folgende allgemeine Formel I auf.

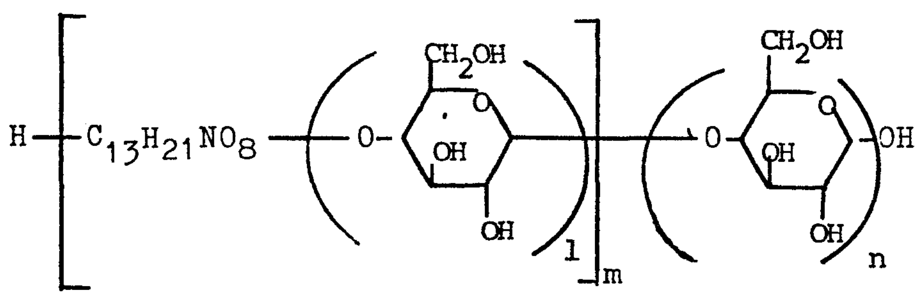

I

in welcher $l$ = 1 oder 2

$m$ = 1,2 oder 3

$n$ = eine ganze Zahl von 1 bis 20 bedeuten.

Sie besitzen basischen Charakter sowie reduzierende Eigenschaften.

Die Erfindung betrifft insbesondere die Pseudooligosaccharide der Formel I, worin $l = 1$, $m = 1$ oder 2 und $n$ = 1,2,3 oder 4 bedeuten. Besonders bevorzugt sind Verbindungen der Formel I, worin

l = 1, m = 2 und n = 1 ist: $C_{44} H_{74} N_2 O_{32}$ Mol.-Gew. 1142 (W-46 A);
l = 1, m = 2 und n = 2 ist: $C_{50} H_{84} N_2 O_{37}$; Mol.-Gew. 1304 (W-46 B);
l = 1, m = 2 und n = 3 ist: $C_{56} H_{94} N_2 O_{42}$; Mol.Gew. 1466, (W-46 C).

sowie deren physiologisch verträgliche Salze mit Säuren. Die Pseudooligosaccharide der Formel I werden in den folgenden Ausführungen auch als die Inhibitoren W-46 bzw. W-46 A, B, und C bezeichnet. Die Verbindungen werden entweder als Gemisch oder als einzelne Verbindungen isoliert.

Die Erfindung betrifft ferner ein Verfahren zur Herstellung der Pseudooligosaccharide der Formel I, pharmazeutische Präparate enthaltend Verbindungen der Formel I, sowie die Verwendung als Arzneimittel, Diagnostikum und Reagenz.

Insbesondere betrifft die Erfindung Verfahren zur Herstellung der Inhibitoren W-46 A, B und C, pharmazeutische Präparate enthaltend diese Verbindungen und ihre Verwendung als Arzneimittel, Diagnostikum und Reagenz.

Das Verfahren zur Herstellung der Pseudooligosaccharide der Formel I ist dadurch gekennzeichnet, daß man einen Pseudooligosaccharid der Formel I erzeugenden Streptomyceten in einem Fermentationsmedium in Submersverfahren kultiviert, die Inhibitoren aus dem Mycel oder dem Kulturfiltrat in an sich bekannter Weise isoliert und reinigt. Von den Streptomyceten ist Streptomyces galbus subsp. FH 1716 geeignet. Dieser Stamm ist bei der Deutschen Sammlung von Mikroorganismen (DSM) unter der

Registrier-Nr. DSM 3007 hinterlegt worden. Es können aber auch die Varianten und Mutanten dieses Stammes zur Gewinnung der Inhibitoren W-46 eingesetzt werden.

Die taxonomischen Eigenschaften des Streptomyces galbus subsp. FH 1716, DSM 3007 entsprechen der Beschreibung von Streptomyces galbus nach dem Bergey's Manual of Determinative Bacteriology, 8. Auflage, Verlag Williams & Wilkins Corp. Baltimore, 1974. Unterschiede zu den beschriebenen Stämmen bestehen in einigen physiologischen Merkmalen. Als Vergleichs- und Referenzstamm ist hierbei der Streptomyces galbus DSM 40480 gewählt worden, die Merkmale sind in der folgenden Tabelle 1 gegenübergestellt.

Tabelle 1

| Kohlenstoffverwertung * der Stämme | I<br>Str. galbus<br>FH 1716 | II<br>Str. galbus<br>DSM 40480 |
|---|---|---|
| Acabinose | + | (+) |
| Xylose | (+) | + |
| Rhamnose | - | - |
| Raffinose | - | (+) |
| Mannose | + | + |
| Inosit | - | + |
| Stärke | - | (+) |
| p-Hydroxybenzoesäure | (+) | + |
| Oxalat | (+) | - |
| Malonat | - | (+) |
| Lactat | - | (+) |
| Gluconat | - | (+) |

* +: gute Verwertung, (+): fragliche Verwertung, -: keine Verwertung

Streptomyces galbus-Stämme mit physiologischen Merkmalen der Spalte I, wie in Tab. 1 wiedergegeben, sind in der Literatur bis jetzt noch nicht beschrieben. Folglich ist der Stamm Streptomyces galbus subsp. FH 1716, DSM 3007 neu. Die Erfindung betrifft daher auch Streptomyces galbus FH 1716, DSM 3007.

Bei der Gewinnung der Inhibitoren W-46 geht man zweckmäßig wie folgt vor:

Streptomyces galbus FH 1716 wird in einem wäßrigen Nährmedium unter submersen und vorzugsweise aeroben Bedingungen gezüchtet, bis man eine ausreichende Konzentration der Inhibitoren W-46 erhält. Das Nährmedium enthält einerseits Kohlenstoffquellen, wie beispielsweise Kohlenhydrate, andererseits Stickstoffquellen, wozu geeignete Stickstoffverbindungen zählen, wie z.B. proteinhaltige Materialien. Bevorzugte Kohlenstoff liefernde Verbindungen sind Glucose, Saccharose, Glycerin, Malzextrakt, Stärke, Öle, Fette und dergleichen. Bevorzugte Stickstoff liefernde Substanzen sind beispielsweise Maisquellwasser, Hefeextrakte, Sojamehl, Fischmehl, Magermilchpulver, angedautes Casein oder Fleischextrakt. Es können auch sogenannte "synthetische" Nährlösungen verwendet werden. Es kann weiterhin nützlich sein, Spurenelemente, wie z.B. Zink, Magnesium, Eisen, Kobalt oder Mangan dem Fermentationsmedium zuzugeben.

Die Fermentation, die zur Bildung der Inhibitoren W-46 führt, kann in einem weiten Temperaturbereich durchgeführt werden. Beispielsweise wird sie bei Temperaturen zwischen 10 und 40°C, vorzugsweise zwischen ungefähr 20 und 35°C durchgeführt. Den pH-Wert des Mediums hält man ebenfalls bei Werten, die dem Wachstum der Mikroorganismen förderlich sind, beispielsweise bei pH-Werten zwischen 4,0 und 10,0, vorzugsweise zwischen 6,0 und 9,0. In Abhängigkeit vom Nährmedium, wie z.B. seiner qualitativen und quantitativen Zusammensetzung und den Fermentationsbedingungen, wie z.B. Belüftungsrate, Temperatur oder pH-Wert, erfolgt die Bildung der Inhibitoren W-46 in der Kulturlösung gewöhnlich nach etwa 1-10 Tagen.

Die Inhibitoren W-46 befinden sich sowohl im Mycel als auch im Kulturfiltrat der Fermentation. Die Hauptmenge des gewünschten Stoffwechselproduktes W-46 ist im allgemeinen im Kulturfiltrat zu finden. Deshalb wird vorteilhafterweise die wäßrige Phase vom Mycel, beispielsweise durch Filtration oder Zentrifugation abgetrennt und das gewünschte Produkt aus den jeweiligen Phasen durch an sich bekannte Verfahren isoliert und gereinigt. Hierzu eignet sich eine große Zahl von Verfahren, wie beispielsweise Chromatographie an Ionenaustauschern, Molekularsieben oder Adsorptionsharzen, Lösungsmittel- bzw. Salzfällungen, Ultrafiltration, Craig-Verteilung u.a.

Ein bevorzugtes Verfahren zur Gewinnung der Komponenten W-46 A, B, bzw. C, besteht darin, daß man die Inhibitoren aus dem Kulturfiltrat an ein geeignetes Harz z.B. auf Polystyrolbasis adsorbiet, dieses beladene Harz abtrennt und die genannten Inhibitoren durch Elution mit geeigneten Pufferlösungen wie z.B. Phosphat- oder Naacetat-Pufferlösung oder mit ggfs. wasserhaltigen organischen Lösungsmitteln, wie z.B. Methanol, Ethanol, Aceton, vorzugsweise aber mit wäßrigem Isopropanol, isoliert. Die inhibitorhaltigen Eluate konzentriert man durch Ultrafiltration in bekannter Weise, wobei gleichzeitig eine Entsalzung vorgenommen wird. Die ionenarme wäßrige Lösung der erwähnten Inhibitoren wird dann auf einer Ionenaustauscher-Säule chromatograhisch in an sich bekannter Weise aufgetrennt. Vorzugsweise verwendet man

stark oder schwach saure Kationenaustauscher z.B. auf Styrol-Divinylbenzol-Copolymerisatbasis, welche als funktionelle Gruppen $-SO_3H$ oder -COOH Gruppen tragen ( ®Dowex 50 W), ( ®Amberlite CG 120), oder auf Basis modifizierter Sulfopropyl-cellulose (SP- ®Sephadex) als Ionenaustauscher, brauchbar sind aber auch eine große Anzahl anderer käuflicher Kationenaustauscher. Der letzte Schritt der Isolierung ist die Anwendung eines Molekularsiebes, z.B. auf Polyacrylamid-Gel-Basis ( ® Biogel P-6) oder auf Basis modifizierter Cellulose ( ®Sephadex). Die resultierenden wäßrigen Lösungen des reinen Materials werden dann z.B. durch Lyophilisierung getrocknet. Die spezifische Aktivität beträgt $4 \times 10^4$ α-Amylaseinhibitoreinheiten per mg Festsubstanz.

Die, nach dem genannten Verfahren gewonnene Substanz ist zwar im wesentlichen frei von Verunreinigungen, muß aber nicht chemisch einheitlich sein. Durch erneute Ionenaustauscherchromatographie z.B. an SP-Sephadex, Molekularsiebchromatogrphie, HPLC-Trennung z.B. an $NH_2$-Gruppentragenden Reversed-Phase-Trägermaterial ( ® Lichrosorb $NH_2$-Träger) mit Acetonitril-Wasser (3:1)-Mischungen und durch ähnliche allgemeinübiche Verfahren kann bei den Produkten der einzelnen Fermentationschargen eine Auftrennung in biologisch wirksame Komponenten möglich sein. Den drei hauptsächlich vorkommenden Komponenten sind die Namen α Amylase-Inhibitor W-46 A, W-46 B und W-46 C gegeben worden. Neben diesen Verbindungen kommen noch weitere inhibitorisch wirksame Pseudooligosaccharide vor.

Die reinen Inhibitoren W-46 sind farblose, amorphe Pseudooligosaccharide. Sie enthalten Stickstoff und haben schwach basischen Charakter. So wandern die Inhibitoren W-46 in der Hochspannungselektrophorse in sauren Puffern, wie z.B. wäßrigen Ameisensäure/Essigsäure Mischungen als Kationen in Richtung Kathode. Die erfindungsgemäßen Substanzen beinhalten Glukose in gebundener Form: durch ihre saure Hydrolyse entsteht Glucose neben anderen, zu meist stickstoffhaltigen Spalt-Produkten. Es ist weiter kennzeichnend für die Inhibitoren W-46, daß sie reduzierende Eigenschaft besitzen, die, wie in der Zuckerchemie üblich, z.B. mit Triphenyltetrazoliumchlorid (TTC) nachgewiesen werden kann.

In der Literatur sind bereits mehrere α-Glukosidaseinhibitoren mit Pseudooligosaccharidcharakter beschrieben worden. E. Truscheit et al., Angew. Chem. 93, Seite 738-755 (1981), T. Tajiri et al., Agric. Biol. Chem. 47, Seite 671-679 (1983), K. Yokose et al.,J. Antibiotics, 36, Seite 1157-1175 (1983).

Durch die allgemeine Formel I aber auch z.T. durch die reduzierende Eigenschaft unterscheiden sich die erfindungsgemäßen Inhibitoren W-46 von allen bekannten α-Glukosidaseinhibitoren, somit liegen hier neue Substanzen vor. Sie zeichnen sich durch eine geringe Polarität aus und sind mikrobiologisch in guten Ausbeuten zu gewinnen.

Die Eigenschaften der erfindungsgemäßen Inhibitoren sind im Hinblick auf die Anwendung als Therapeutikum gegen Diabetes und Prädiabetes sowie Adipositas und Unterstützung von Diät interessant. Aufgrund ihrer Eigenschaften sind sie auch als Reagenz für diagnostische Zwecke wertvoll.

Stärkehaltige Nahrungs- und Genußmittel führen bei Tier und Mensch zu einem Anstieg des Blutzuckers und dadurch auch zu einer vermehrten Insulinsekretion des Pankreas. Die Hyperglykämie kommt durch die Aufspaltung der Stärke im Verdauungstrakt unter Einwirkung von Amylase und Maltase zu Glukose zustande.

Bei Diabetikern ist die Hyperglykämie besonders ausgeprägt und langanhaltend.

Die alimentäre Hyperglykämie sowie die Hyperinsulinämie nach Stärkeaufnahme läßt sich durch die erfindungsgemäßen Amylase-Inhibitoren insbesondere durch W-46 A, B, und C, vermindern. Diese Wirkung ist dosisabhängig. Die erfindungsgemäßen Amylase-Inhibitoren lassen sich daher als Therapeutikum einsetzen bei Diabetes, Prädiabetes und Adipositas sowie zur Unterstützung von Diät. Zu diesem Zweck empfiehlt sich eine orale Verabreichung, insbesondere zu den Mahlzeiten. Die Dosierung, die sich nach dem Gewicht des Patienten sowie dem individuellen Bedarf ausrichten soll, beträgt etwa 5-500 mg pro Dosis, die zweckmäßig zu jeder Mahlzeit genommen wird. Die Dosierung kann jedoch in begründeten Einzelfällen auch darüber oder darunter liegen.

Die erfindungsgemäßen Amylase-Inhibitoren eignen sich insbesondere zur oralen Verabreichung. Sie können als reine Substanz, als ihre physiologisch verträglichen Salze mit Säuren, aber auch in Form einer pharmazeutischen Zubereitung unter Verwendung der üblichen Hilfs- und Trägerstoffe angewandt werden. Auch eine kombinierte Anwendung mit anderen Arzneimitteln wie blutzuckersenkenden oder lipidsenkenden Substanzen kann von Vorteil sein. Da höhermolekulare Saccharide als solche nicht oder nicht nennenswert aus dem Verdauungstrakt resorbiert werden, sind von den erfindungsgemäßen Substanzen keine toxikologisch bedenklichen Nebenwirkungen zu erwarten. Dementsprechend konnten nach oraler Gabe auch hoher Dosen der Amylaseinhibitoren W-46 an Versuchstieren keine auffälligen Symptome erkannt werden. Zur Prüfung der pharmakologischen Wirkung des Amylase-Inhibitors erhielten nüchterne, männliche Wistar-Ratten mit einem Gewicht zwi-

schen 200 und 250 g einen erfindungsgemäßen Hemmstoff W-46 oder ein Gemisch gleichzeitig mit 2 g Stärke pro kg Körpergewicht oral verabreicht. Durch Bestimmung von Blutzuckerkonzentrationen in Blutproben, die vor, während und nach der Applikation des α-Amylaseinhiitors entnommen worden sind, wurde die Wirksamkeit des Präparates bewiesen. Neben der Blutglukoseregulation kann man die erfindungsgemäßen Oligosaccharide auch zur Hemmung der Speichel-α-Amylase verwenden. Dieses Enzym bewirkt die Verdauung der Stärke im Mund und der so gebildete Zucker fördert den Kariesbefall der Zähne. Die erfindungsgemäßen Verbindungen können daher zur Verhütung oder Verminderung der Ausbildung von Karies angewandt werden.

Weiterhin können sie als biochemische Reagenzien sowie als diagnostische Mittel verwendet werden.

Amylasetest

Eine Amylase-Inhibitoreinheit (AIE) ist definiert als die Inhibitormenge, die unter den Testbedingungen zwei Amylase-Einheiten (AE) zu 50 % zu hemmen vermag. Eine Amylase-Einheit ist nach internationaler Übereinkuft die Enzymmenge, die in einer Minute 1 µ äquivalent glucosidischer Bindungen in der Stärke spaltet. Die µVal gespaltener glucosidischer Bindungen werden als µVal reduzierender Zucker photomerisch mit Dinitrosalizylsäure bestimmt. Die Angaben sind als µMole Maltose berechnet, die anhand einer Maltose-Eichgeraden ermittelt werden.

Die Tests werden folgendermaßen durchgeführt:

α-Amylase aus Schweinepankreas und die zu testenden Lösungen werden gemeinsam in 1,0 ml 20 mM Phosphatpuffer, pH 6,9, + 10mM NaCl 10-20 min. bei 37°C vorinkubiert. Die

enzymatische Reaktion wird durch Zugabe von 1,0 ml löslicher Stärke (0,25 % in dem angegebenen Puffer) nach Zulkowski gestartet. Nach genau 10 min. wird die Reaktion mit 2,0 ml Dinitrosalizylsäure-Farbreagenz (nach Boehringer Mannheim: Biochemica-Information II) abgestoppt und zur Farbentwicklung 5 min. im siedenden Wasserbad erhitzt. Nach dem Abkühlen wird die Extinktion bei 546 nm gegen den Reagentienleerwert gemessen. Die 50%ige Hemmung wird im Vergleich zur ungehemmten Enzymreaktion durch Einsatz verschiedener Inhibitormengen graphisch mittels der Wahrscheinlichkeitsauftragung bestimmt.

Beispiel 1

Zur Gewinnung von W-46 erfolgte die Impfstoffanzucht (Anzucht des Mikroorganismus) - wie in der mikrobiologischen Praxis üblich - aus einer gefriergetrockneten Dauerform des Organismus Streptomyces galbus, FH 1716 DSM 3007, über Einzelkoloniepassage und Schrägröhrchen. Die für die Fermentation notwendige Massenproduktion an Sporen wurde ebenfalls auf festem Nährboden in Roux-Flaschen durchgeführt.

Agarmedium für Platte, Schrägröhrchen und Roux-Flasche:

| | |
|---|---|
| Dextrin | 15,0 g/l |
| Rohrzucker | 3,0 g/l |
| Fleischextrakt | 1,0 g/l |
| Hefeextrakt | 2,0 g/l |
| Kochsalz | 0,5 g/l |
| $K_2HPO_4$ | 0,5 g/l |
| $FeSO_4 \times 7\ H_2O$ | 0,01 g/l |
| Agar-Agar | 2,0 g/l |
| pH-Wert | 7,3 |

| | | | |
|---|---|---|---|
| Sterilisation | bei | 120°C | 20 min. |
| Inkubation | bei | 30°C | 9 Tage. |

Das beimpfte Röhrchen und die Roux-Flasche wurden 7 Tage bei 30°C bebrütet und danach bei +4°C gehalten. Die Sporen wurden mit 10 ml sterilisiertem Aqua dest. oder physiologischer Kochsalzlösung von dem festen Nährboden abgeschwemmt. 5 ml der Suspension dienten zur Beimpfung eines 2 000 ml Erlenmeyerkolbens, der mit 500 ml sterilisierter wäßriger Nährlösung mit einem pH-Wert von 7,7 und folgender Zusammensetzung beschickt war (Angaben in Gew.-%).

1,00 % Glukose
0,40 % Caseinpepton
0,40 % Fleischextrakt
0,25 % NaCl
0,05 % Hefeextrakt
0,05 % Leberpulver.

Der Kolben wurde bei 220 Upm 48 Stunden bei +30°C auf einer Schüttelmaschine geschüttelt. Danach wurde diese Vorkultur in ein 12 l-Fermenter überführt, der mit 9 l sterilisierter wäßriger Nährlösung beschickt war und einen pH-Wert von 7,4 hatte. Die Zusammensetzung der Nährlösung für die Hauptkultur war folgende (Angaben in Gewichts-%).

2,0 % Fleischextrakt
2,0 % Malzextrakt
1,0 % Calciumcarbonat
0,1 % Antischaummittel,
ad 100 % Wasser.

Die Hauptkultur wurde bei 28°C, 2 Tage mit 850Upm gerührt, die Luftzufuhr betrug 420 l pro Stunde. Nach 18,

24, 30, 36, 40, 44 und 48 Stunden wurde der Gehalt an α-Amylaseinhibitor gemäß der Vorschrift von R. Bender et al., Anal. Biochem, 137, 307-312 (1984) bestimmt. Der Stamm Str. galbus FH 1716 lieferte unter den beschriebenen Versuchs- und Kulturbedingungen durchschnittlich 5 x $10^3$ AIE/ml beieinem End-pH von 9,0.

Beispiel 2

8 l Fermentationslösung gemäß Beispiel 1 wurden mit Hilfe einer Zentrifuge von der Zellmasse befreit und die klare flüssige Phase wurde auf pH 9,5 eingestellt. Anschließend trug man die Lösung auf eine 0,8 l Polystyrol-Adsorptionsharz (Diaion® HP-20) beinhaltende Säule auf, wusch mit 1,5 l Wasser nach und eluierte mit Wasser, dem steigende Mengen Isopropanol hinzugefügt worden war. Die Mischung, die 10 % Isopropanol enthielt, löste den Inhibitor W-46 von der Säule. Diese aktiven Eluate (1,2 l) wurden durch Ultrafiltration konzentriert und unter Wasserzugabe sowie weitere Ultrafiltration solange entsalzt, bis das Retenat keine nachweisbaren Salze mehr enthielt. Das resultierende Konzentrat (0,2 l) trennte man auf Sulfopropyl modif. Cellulose (SP-Sephadex®,die in die Säureform ($H^+$-Form) überführt war. Durch Anlegen eines Ammonium-acetatgradienten, pH 5 (0-0,5 molar), wurden die die α-Amylase-hemmende Aktivität enthaltenden Fraktionen eluiert. Die entsprechenden Fraktionen wurden in Ultrafiltrationszellen ( ®Amicon) konzentriert und entsalzt. Die Endreinigung geschah an Polyacrylamid-Gel (Biogel® P-6) mit reinem Wasser als Laufmittel. Die W-46-haltigen Fraktionen dieser Säule wurden gesammelt und gefriergetrocknet. Es resultierten 1,5 g eines hellbeigen amorphen Pulvers mit einer α-Amylase-hemmenden Wirksamkeit von 4 x $10^4$ AIE pro mg Substanz. In Figur 1 ist das IR-Spektrum (in KBr), in Figur 2, das NMR-Spektrum (in DMSO) wiedergegeben.

Beispiel 3

100 mg des Inhibitoren-Gemisches W-46 - gewonnen nach Beispiel 2 - wurden in 0,01 % Phosphatpuffer, pH 7,8, gelöst und auf 150 g Lichrosorb RP 18-Träger in einer Stahlsäule unter HPLC-Bedingungen getrennt. Als Elutionsmittel diente hierbei 95 % 0,01 %iger Phosphatpuffer, pH 7,8, mit 5 % Acetonitril, die Detektion des Eluats erfolgte durch UV-Absorptionsmessung bei 210 nm. Nach 4, 5,8 und 7,5 Minuten wurde inhibitorisch wirksames Material getrennt von der Säule gewonnen. Diese Fraktionen wurden eingeengt, entsalzt und gefriergetrocknet. Ihre Analyse ergab:

Peak I (nach 4 Minuten HPLC); C: 45,8 %, H: 6,4 %, N: 1,9 %, O: 46,0 %. Durch FAB-Massenspektrometrie wurde ein M + $H^+$-Peak von 1467 gefunden (W-46 C).

Peak II (nach 5,8 Minuten HPLC); C: 46,1 %, H: 6,5 %, N: 2,1 %, O: 45,4 %. FAB-Massenspektrometrie (FAB-MS), M + $H^+$-Peak: 1305 (W-46 B)

Peak III (nach 7,5 Minuten HPLC); C: 46,3 %, H: 6,5 %, N: 2,5 %, O: 44,8 %. FAB-MS, M + $H^+$-Peak: 1143 (W-46 A).

Beispiel 4

100 mg des nach Beispiel 2 gewonnenen Materials wurden 0,5 ml Wasser gelöst, bei 0°C mit Salzsäure auf pH 1 gestellt und mit 5 ml Aceton versetzt. Der resultierende Niederschlag wurde durch Zentrifugieren gesammelt, in Wasser aufgenommenund gefriergetrocknet. Man erhielt das Hydrochlorid des Inhibitoren-Gemisches W-46.

Beispiel 5

Veränderungen des Blutglucoseanstiegs der stärkebelasteten Ratte in Abhängigkeit von der oral verabreichten Dosis des Inhibitoren-Gemisches W-46.

Methode:

Versuchstiere waren 45 männliche Albino-Ratten. Die Tiere hatten 18 h vor und während des Versuches keinen Zugang zum Futter. Je 8 - 10 Tiere erhielten 0.3, 0.6 und 1.0 mg/kg Inhibitor W-46 gemäß Beispiel 2 zusammen mit 2 g/kg Stärke in Leitungswasser suspendiert mittels Schlundsonde p.o.. Das Applikationsvolumen betrug 1 mg/100 g Körpergewicht. 18 Kontrolltiere erhielten nur Stärkesuspension. Unmittelbar vor sowie 0.5, 1, 2, 3 und 5 h nach Behandlung wurden je 10 µl Blut entnommen und die Blutglucose enzymatisch bestimmt.

Ergebnisse:

Das Inhibitoren-Gemisch W-46 verminderte dosisabhängig den postprandialen Blutglucoseanstieg nach Stärkebelastung. 1h nach Behandlung bewirkten 0.3, 0.6 und 1 mg/kg Inhibitor W-46 eine mindestens 16, 23 und 25%ige Verringerung des Blutzuckeranstieges gegeüber den Kontrollwerten. Die Dosisabhängigkeit ist statistisch gesichert. Die Grenzwertdosis für den mittleren prozentualen Blutglucoseanstieg über 3 h beträgt 0.4 mg/kg.

PATENTANSPRÜCHE:

1. Psendooligosaccharide der Formel I

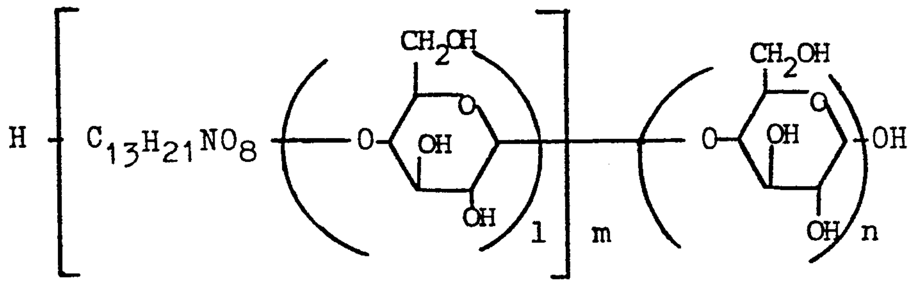

I

in welcher $l$ = 1 oder 2
$m$ = 1,2 oder 3
$n$ = eine ganze Zahl von 1 bis 20 bedeuten.

und deren physiologisch verträgliche Salze mit Säuren.

2. Pseudooligosaccharide gemäß Anspruch 1, dadurch gekennzeichnet, daß in Formel I $l$ = 1 $m$ = 1 oder 2 und $n$ = 1,2,3 oder 4 bedeuten.

3. Verfahren zur Herstellung von Pseudooligosacchariden gemäß Anspruch 1, dadurch gekennzeichnet, daß man einen Pseudooligosaccharide der Formel I erzeugenden Streptomyceten in einem Fermentationsmedium im Submersverfahren kultiviert, die Pseudooligosaccharide aus dem Mycel oder Kulturfiltrat in an sich bekannter Weise isoliert und reinigt und gegebenenfalls in ein physiologisch verträgliches Salz mit einer Säure überführt.

4. Verfahren gemäß Anspruch 3, dadurch gekennzeichnet, daß man Streptomyces galbus subsp. FH 1716 kultiviert und aus dem Kulturfiltrat die Pseudooligosaccharide isoliert und reinigt.

5. Verfahren gemäß Anspruch 3, dadurch gekennzeichnet, daß man ein Pseudooligosaccharid der Formel I, worin l = 1 m = 1 oder 2 und n = 1,2,3 oder 4 bedeutet, isoliert.

6. Pharmazeutisches Präparat gekennzeichnet durch einen Gehalt an einem Pseudooligosaccharid gemäß Anspruch 1.

7. Pharmazeutisches Präparat dadurch gekennzeichnet, daß es ein Pseudooligosaccharid der Formel I worin l = 1 m = 1 oder 2 und n = 1,2,3 oder 4 bedeuten, enthält.

8. Verwendung von Pseudooligosacchariden gemäß Anspruch 1 zur Hemmung der α-Glukosidase.

9. Verwendung von Pseudooligosacchariden gemäß Anspruch 1 zur Behandlung von Diabetes, Prädiabetes und Adipositas.

10. Verwendung von Pseudooligosacchariden gemäß Anspruch 1 als Diagnostikum, Reagenz oder Prophylaktikum gegen Karies.

11. Streptomyces galbus subsp. FH 1716, DSM 3007.

Patentansprüche für Österreich

1. Verfahren zur Herstellung von Pseudooligosacchariden der Formel I

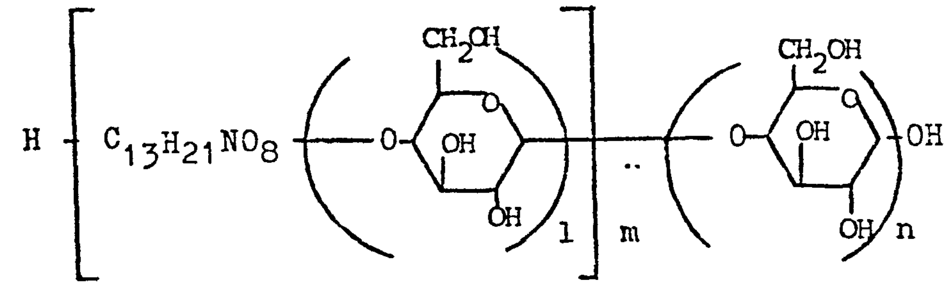

I

in welcher l = 1 oder 2
m = 1,2 oder 3
n = eine ganze Zahl von 1 bis 20 bedeuten

und von deren physiologisch verträglichen Salzen mit Säuren, dadurch gekennzeichnet, daß man einen Pseudooligosaccharide der Formel I erzeugenden Streptomyceten in einem Fermentationsmedium im Submersverfahren kultiviert, die Pseudooligosaccharide aus dem Mycel oder Kulturfiltrat in an sich bekannter Weise isoliert und reinigt und gegebenenfalls in ein physiologisch verträgliches Salz mit einer Säure überführt.

2. Verfahren gemäß Anspruch 1, dadurch gekennzeichnet, daß man Streptomyces galbus subsp. FH 1716 kultiviert und aus dem Kulturfiltrat die Pseudooligosaccharide isoliert und reinigt.

3. Verfahren gemäß Anspruch 1, dadurch gekennzeichnet, daß man ein Pseudooligosaccharid der Formel I, worin l = 1, m = 1 oder 2 und n = 1,2,3 oder 4 bedeutet, isoliert.

4. Verfahren zur Herstellung eines pharmazeutischen Präparats, dadurch gekennzeichnet, daß man ein Pseudooligosaccharid gemäß Anspruch 1, Formel I, oder ein physiologisch verträgliches Salz dieser Verbindung in eine geeignete Applikationsform überführt.

5. Verfahren gemäß Anspruch 4, dadurch gekennzeichnet, daß man ein Pseudooligosaccharid der Formel I worin l = 1, m = 1 oder 2 und n = 1,2,3 oder 4 bedeuten, verwendet.

6. Verwendung von Pseudooligosacchariden gemäß Anspruch 1 zur Hemmung der α-Glukosidase.

7. Verwendung von Pseudooligosacchariden gemäß Anspruch 1 als Diagnostikum, Reagenz oder Prophylaktikum gegen Karies.

8. Streptomyces galbus subsp. FH 1716, DSM 3007.

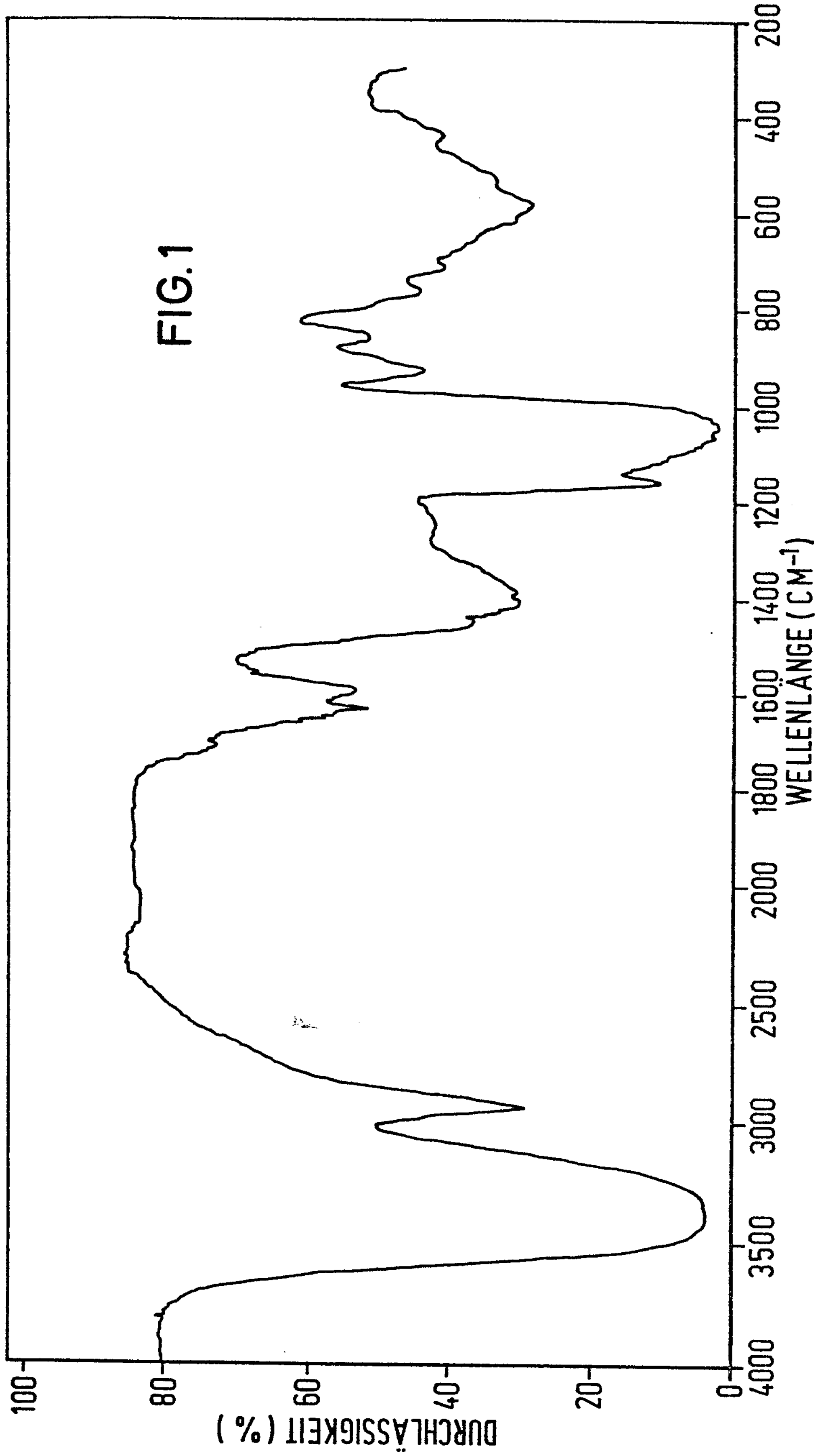
DURCHLÄSSIGKEIT ( % )
100
80
60
40
20
0
4000
3500
3000
2500
2000
1800
1600
1400
1200
1000
800
600
400
200
WELLENLÄNGE ( CM⁻¹ )

FIG.1

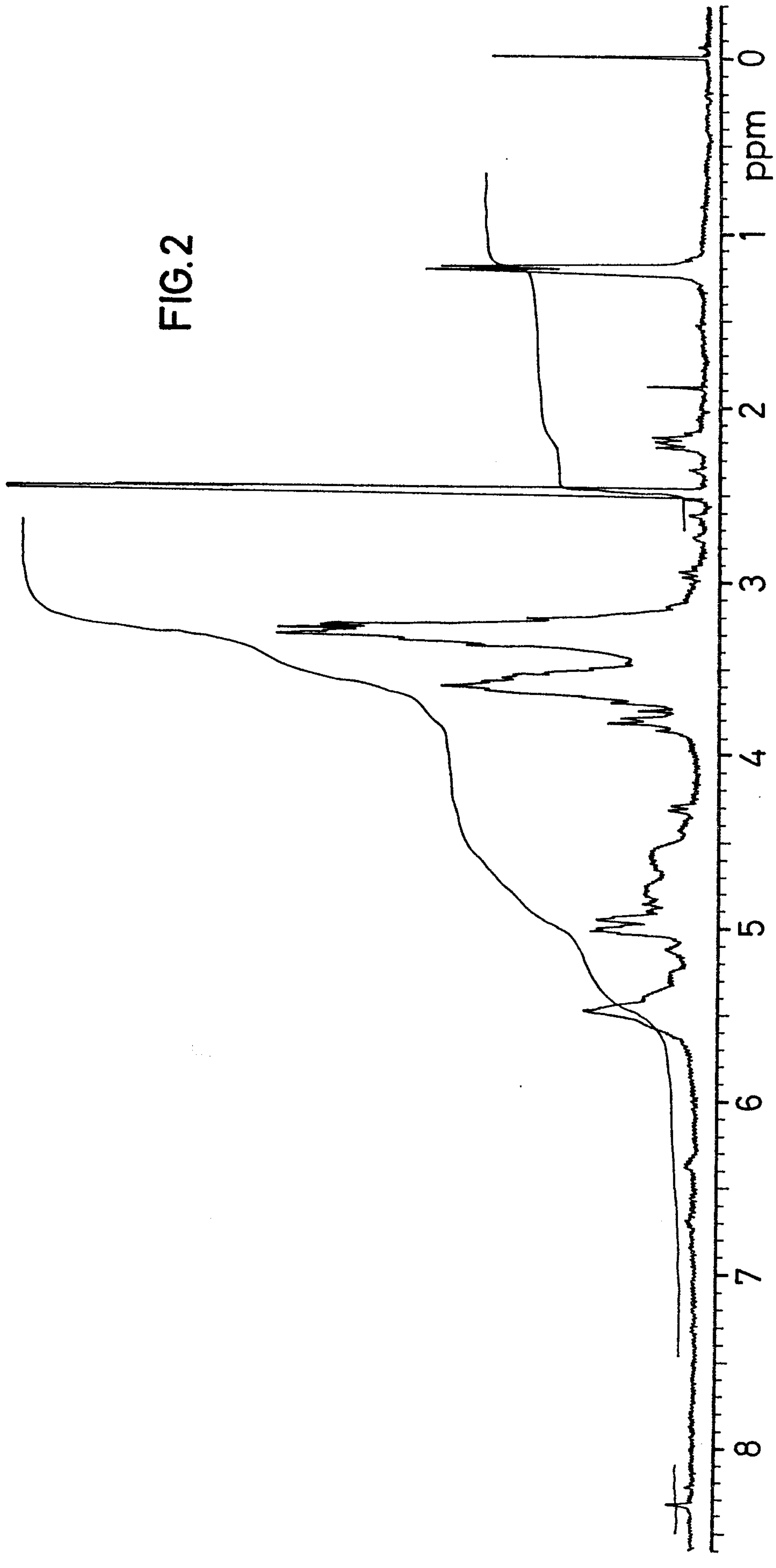


FIG.2